# EUROPÄISCHE PATENTSCHRIFT

(11) **EP 1 097 878 B1**
(45) Veröffentlichungstag und Bekanntmachung des Hinweises auf die Patenterteilung: **06.08.2008**
(21) Anmeldenummer: 99124744.6
(22) Anmeldetag: 13.12.1999
(51) Int. Cl.: B65D 65/02, A61F 13/15

(54) **Bahnförmiges Verpackungsmaterial**
Web packaging material
Bande de matériau d'emballage

(30) Priorität: 02.11.1999 DE 19952569
(43) Veröffentlichungstag der Anmeldung: 09.05.2001
(73) Patentinhaber: 4P Folie Forchheim GmbH, 91301 Forchheim (DE)
(72) Erfinder: Schmidt, Werner, 96129 Strullendorf (DE)
(74) Vertreter: Hutzelmann, Gerhard

(56) Entgegenhaltungen:
- WO-A-98/53781
- WO-A-99/60965
- DE-A- 3 541 753
- GB-A- 2 298 627
- US-A- 5 333 753

## Beschreibung

Die Erfindung bezieht sich auf ein bahnförmiges Verpackungsmaterial zum wenigstens annähernd allseitigen Einschlagen eines zu verpackenden Gegenstandes, der einseitig mit einem Kleber versehen ist.

Vor dem Einschlagen muß die Kleber-Schicht mit einem Abschnitt mit Releaseeigenschaften abgedeckt werden, damit ihre Klebkraft bis zur eigentlichen Anwendung erhalten bleibt. Der Gegenstand wird dann in das Verpackungsmaterial eingeschlagen, wobei die Ränder durch siegeln, kleben, krümpern od.dgl. miteinander verbunden werden um eine wenigstens annähernd dichte Verpackung zu erzielen.

Diese Art der Verpackung ist sehr aufwendig.

Deswegen wurden im Stand der Technik bereits Lösungen vorgeschlagen, die sich dieses Problems annehmen.

Aus der DE-A-35 41 753 ist es beispielsweise bekannt, ein bahnförmiges Verpackungsmaterial vorzusehen, welches zum wenigstens annähernd allseitigen Einschlagen eines zu verpackenden Gegenstandes dient, der einseitig mit einem Kleber versehen ist, wobei eine Seite des Verpackungsmaterials mit mit Releasematerial beschichteten Release-Abschnitten versehen ist, neben denen Abschnitte vorgesehen sind, die frei von Releasematerial sind.

Hiervon ausgehend liegt der Erfindung die Aufgabe zugrunde, ein bahnförmiges Verpackungsmaterial der genannten Art so zu verbessern, daß das Herstellen der Verpackung einfacher zu realisieren ist.

Diese Aufgabe wird erfindungsgemäß dadurch gelöst, daß wenigstens drei Längsstreifen mit Releasebeschichtung vorgesehen sind, wobei jeweils zwischen zwei benachbarten Längsstreifen mit Releasebeschichtung ein Längsstreifen, der frei von Releasematerial ist, angeordnet ist und sämtliche Längsstreifen parallel zueinander und in der gemeinsamen Ebene des Verpackungsmaterials angeordnet sind.

Der zu verpackende Gegenstand wird dabei auf der Releaseschicht mit seinem Kleber festgelegt und kann dann weiter eingepackt werden.

Zudem ist es möglich im Bereich dieses freien Randstreifens eine Siegelnaht, eine Klebenaht oder eine Krümpernaht vorzusehen.

Desweiteren läßt sich auch eine sehr dichte und stabile Verpackung herstellen, die an beiden Längsrändern mit Verschlußnähten versehen ist und nur dort Releasebeschichtungen aufweist, wo sie benötigt werden.

Gegenstände mit segmentierten Kleberschichten lassen sich auch sehr effektiv verpacken.

Eine weitere vorteilhafte Ausgestaltung der Erfindung liegt darin, daß einzelne Abschnitte mit Releasebeschichtung vorgesehen sind, die entsprechend den zu verpackenden Gegenständen bzw. deren Kleberbeschichtung angeordnet sind.

Damit ist eine sehr präzise Anpassung der Releasebeschichtung an die Notwendigkeit der Kleberabdeckung des zu verpackenden Gegenstandes gewährleistet.

Als sehr vorteilhaft hat es sich erwiesen, wenn gemäß einer weiteren Ausgestaltung der Erfindung die Releasebeschichtung aus Polysiloxanen besteht, die gehärtet sind.

Derartigen Releasebeschichtungen haben sich bereits sehr bewährt.

Eine vorteilhafte Ausgestaltung der Erfindung ist, **dadurch gekennzeichnet, daß** die Verpackungsmaterialbahn aus einer Kunststofffolie besteht, die als Mono- oder Mehrschichtfolie ausgebildet ist und geprägt sein kann.

Auf einer derartigen Verpackungsmaterialbahn lassen sich sowohl die Releaseschicht als auch eine Verbindungsschicht für die Randverbindung sehr gut aufbringen.

Gemäß einer weiteren vorteilhaften Ausgestaltung der Erfindung ist es aber auch möglich, daß die Verpackungsmaterialbahn aus Papier besteht, das vorzugsweise wenigstens einseitig mit Kunststoff beschichtet ist.

Eine weitere vorteilhafte Ausgestaltung der Erfindung liegt darin, daß die Verpackungsmaterialbahn aus einem Vliesstoff besteht, der vorzugsweise wenigstens einseitig mit Kunststoff beschichtet ist.

Sowohl mit Papier als auch mit Vliesstoff lassen sich die vorteilhaften Eigenschaften der Erfindung auf einfache Weise realisieren.

Eine vorteilhafte Ausgestaltung der Erfindung liegt auch darin, daß ein Längsabschnitt der Verpackungsmaterialbahn als Einschlagverpackung für einen einseitig klebend ausgerüsteten Gegenstand vorgesehen ist.

Sehr vorteilhaft ist es auch, wenn gemäß einer weiteren vorteilhaften Ausgestaltung der Erfindung die Anordnung der Releasebeschichtung entsprechend dem zu verpackenden Gegenstand angeordnet ist.

Bei einer erfindungsgemäß zu verpackenden Damenbinde wird die Releasebeschichtung zweckmäßiger Weise nur im mittleren Bereich vorgesehen sein, in dem auch die Kleberbeschichtung der Damenbinde sitzt.

Soll jedoch eine Damenbinde mit seitlich vorstehenden Flügeln verpackt werden, so ist auch in deren Bereich eine Releasebeschichtung vorgesehen, da auch die Flügel mit Kleber versehen sind.

Gemäß der Erfindung kann als zu verpackender Gegenstand auch ein selbstklebend ausgerüstetes Wundpflaster vorgesehen sein.

Bei allen Ausgestaltungen kann gemäß einer weiteren vorteilhaften Weiterbildung der Erfindung vorgesehen sein, daß die von der Releasebeschichtung abgekehrte Seite der Verpackungsmaterialbahn ein Druckbild trägt.

In der Zeichnung ist die Erfindung anhand mehrerer Ausführungsbeispiele veranschaulicht. Dabei zeigt die einzige Figur:

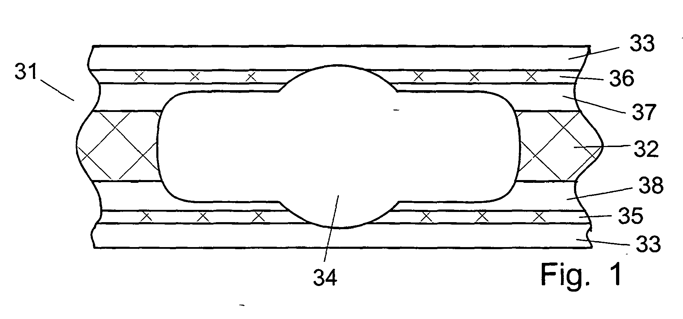

- Fig.1: einen Abschnitt einer Verpackungsmaterialbahn mit drei Längsstreifen mit Releasebeschichtung, die jeweils von Streifen ohne Releasebeschichtung längsseits eingerahmt sind,

Mit 31 ist in Fig.1 eine Verpackungsmaterialbahn bezeichnet, die mit einer Releaseschicht 32 sowie zwei Randstreifen 33 versehen ist, wobei die Randstreifen 33 frei von einer Releasebeschichtung sind. Auf die Releasebeschichtung 32 wird - wie in Fig. 1 dargestellt - ein Gegenstand 34 aufgelegt, der in einen entsprechenden Abschnitt der Verpackungsmaterialbahn eingeschlagen werden soll. Der Gegenstand 34 - beim dargestellten Ausführungsbeispiel eine Damenbinde mit seitlichen Flügeln - ist auf seiner Unterseite mit einem Haftkleber versehen, der zwar auf der Releasebeschichtung 32 klebt aber sich leicht wieder ablösen läßt ohne seine Klebkraft zu verlieren. Zwei seitliche Abschnitte der Verpackungsmaterialbahn werden zum Verpacken der Damenbinde über diese gefaltet und die übereinanderliegenden Randstreifen 33 miteinander versiegelt. Der gegenüberliegende Rand wird dann eingefaltet und die Verpackung in nicht dargestellter Weise geschlossen.

Anstelle einer Versiegelung der Randstreifen ist auch ein Kleben oder eine Verkrümperung möglich.
Die Verpackungsmaterialbahn 31 gemäß Fig.1 dient auch dem Verpacken einer Damenbinde 34 mit Flügeln, bei der die Flügel mit Kleberstellen versehen sind. Die Verpackungsmaterialbahn 31 ist mit drei Längsstreifen 32, 35 und 36 mit Releasebeschichtung versehen, die durch zwei Längsstreifen 37 und 38 voneinander getrennt sind. Diese Längsstreifen 37 und 38 sind genauso wie die beiden Randstreifen 33 frei von einer Releasebeschichtung, so daß zumindest die Randstreifen miteinander verbunden werden können.

Es ist auch denkbar, daß eine derartige Verpackungsmaterialbahn auch zum Verpacken von Wundpflastern dienen kann.

Als Material für die Verpackungsmaterialbahn kommen Kunststoffolien in Frage, die als Monofolien oder als Mehrschichtfolien ausgebildet sind. Darüber hinaus ist es möglich, Papier zu verwenden. Auch Vliesstoffe können für die Verpackungsmaterialbahn verwendet werden. Sowohl die Papierbahn als auch die Vliesbahn können wenigstens einseitig mit Kunststoff beschichtet werden.

Als Releasebeschichtung haben sich Polysiloxane besonders bewährt, wobei sowohl lösemittelhaltige als auch lösemittelfreie Polysiloxane eingesetzt werden können. Die Härtung der Polysiloxane erfolgt entweder thermisch oder durch Strahlenhärtung.

Auf der von der Releaseschicht abgewandten Seite kann die Verpackungsmaterialbahn noch mit einem Druckbild versehen sein.

## Patentansprüche

1. Bahnförmiges Verpackungsmaterial (31) zum wenigstens annähernd allseitigen Einschlagen eines zu verpackenden Gegenstandes (34), der einseitig mit einem Kleber versehen ist, wobei eine Seite des Verpackungsmaterials (31) mit mit Releasematerial beschichteten Release-Abschnitten versehen ist, neben denen Abschnitte vorgesehen sind, die frei von Releasematerial sind, **dadurch gekennzeichnet, daß** beim Einschlagen des zu verpackenden Gegenstandes wenigstens drei Längsstreifen (32,35,36) mit Releasebeschichtung vorgesehen sind, wobei jeweils zwischen zwei benachbarten Längsstreifen mit Releasebeschichtung ein Längsstreifen, der frei von Releasematerial ist, angeordnet ist und sämtliche Längsstreifen parallel zueinander und in der gemeinsamen und durchgehenden Ebene des Verpackungsmaterials angeordnet sind.

2. Bahnförmiges Verpackungsmaterial nach Anspruch 1, **dadurch gekennzeichnet, daß** die Releasebeschichtung aus Polysiloxanen besteht, die gehärtet sind.

3. Bahnförmiges Verpackungsmaterial nach Anspruch 1 oder 2, **dadurch gekennzeichnet, daß** die Verpackungsmaterialbahn (31) aus einer Kunststofffolie besteht, die als Mono- oder Mehrschichtfolie ausgebildet ist und geprägt sein kann.

4. Bahnförmiges Verpackungsmaterial nach einem der Ansprüche 1 bis 3, **dadurch gekennzeichnet, daß** die Verpackungsmaterialbahn (31) aus Papier besteht, das vorzugsweise wenigstens einseitig mit Kunststoff beschichtet ist.

5. Bahnförmiges Verpackungsmaterial nach einem der Ansprüche 1 bis 3, **dadurch gekennzeichnet, daß** die Verpackungsmaterialbahn (31) aus einem Vliesstoff besteht, der vorzugsweise wenigstens einseitig mit Kunststoff beschichtet ist.

6. Bahnförmiges Verpackungsmaterial nach einem der vorhergehenden Ansprüche, **dadurch gekennzeichnet, daß** die Anordnung der Releasebeschichtung (32,35,36) entsprechend den Anforderungen des zu verpackenden Gegenstandes (34) ausgebildet ist.

7. Bahnförmiges Verpackungsmaterial nach Anspruch 6, **dadurch gekennzeichnet, daß** die Anordnung der Releasebeschichtung (32,35,36) zur Aufnahme einer Damenbinde (34) mit seitlich vorstehenden Flügeln ausgebildet ist.

8. Bahnförmiges Verpackungsmaterial nach Anspruch 6, **dadurch gekennzeichnet, daß** die Anordnung der Releasebeschichtung (32,35,36) zur Aufnahme eines selbstklebend ausgerüsteten Wundpflasters ausgebildet ist.

9. Bahnförmiges Verpackungsmaterial nach einem der vorhergehenden Ansprüche, **dadurch gekennzeichnet, daß** die von der Releasebeschichtung abgekehrte Seite der Verpackungsmaterialbahn ein Druckbild trägt.

## Claims

1. Web-like packaging material (31) to wrap an object (34) on at least approximately all sides that requires packaging and is provided with adhesive on one side, where one side of the packaging material (31) is provided with release sections coated with release material, next to which sections are provided that have no release material, **wherein** at least three longitudinal sections (32, 35, 36) with a release coating are provided when wrapping the object that requires packaging, where one longitudinal strip that has no release material is located between each pair of adjacent longitudinal strips with release coating and where all the longitudinal strips are located parallel to each other and in the same, uninterrupted plane of the packaging material.

2. Web-like packaging material according to claim 1, **wherein** the release coating consists of polysiloxanes that are cured.

3. Web-like packaging material according to claim 1 or 2, **wherein** the web of packaging material (31) consists of a plastic film that takes the form of a mono- or multilayer film and can be embossed.

4. Web-like packaging material according to one of claims 1 to 3, **wherein** the web of packaging material (31) consists of paper that is preferably coated with plastic on at least one side.

5. Web-like packaging material according to one of claims 1 to 3, **wherein** the web of packaging material (31) consists of a nonwoven fabric that is preferably coated with plastic on at least one side.

6. Web-like packaging material according to one of the previous claims, **wherein** the release coating (32, 35, 36) is located according to the requirements of the object (34) that requires packaging.

7. Web-like packaging material according to claim 6, **wherein** the release coating (32, 35, 36) is located to accommodate a sanitary towel (34) with projecting wings at the sides.

8. Web-like packaging material according to claim 6, **wherein** the release coating (32, 35, 36) is located to accommodate a self-adhesive sticking plaster.

9. Web-like packaging material according to one of the previous claims, **wherein** the side of the web of packaging material opposite the side with the release coating has a print motif.

## Revendications

1. Matériau d'emballage en forme de bande (31) pour envelopper au moins approximativement de tous côtés un objet à emballer (34) qui est revêtu de colle sur une face, une face du matériau d'emballage (31) étant dotée de segments anti-adhérents revêtus d'agent anti-adhérent à côté desquels sont prévus des segments exempts d'agent anti-adhérent, **caractérisé en ce que** lors de l'emballage de l'objet à emballer, au moins trois bandes longitudinales (32, 35, 36) revêtues d'agent anti-adhérent sont prévues, sachant qu'entre deux bandes longitudinales munies d'agent anti-adhérent est chaque fois prévue une bande longitudinale exempte d'agent anti-adhérent et que toutes les bandes longitudinales sont disposées parallèlement entre elles et situées dans le plan commun et ininterrompu du matériau d'emballage.

2. Matériau d'emballage en forme de bande selon la revendication 1, **caractérisé en ce que** le revêtement anti-adhérent est composé de polysiloxanes durcis.

3. Matériau d'emballage en forme de bande selon la revendication 1 ou 2, **caractérisé en ce que** la bande de matériau d'emballage (31) est composée d'une feuille de matière plastique qui est réalisée en tant que feuille monocouche ou multicouche et qui peut être gaufrée.

4. Matériau d'emballage en forme de bande selon l'une des revendications 1 à 3, **caractérisé en ce que** la bande de matériau d'emballage (31) est constituée de papier qui est revêtu de matière plastique de préférence sur au moins une face.

5. Matériau d'emballage en forme de bande selon l'une des revendications 1 à 3, **caractérisé en ce que** la bande de matériau d'emballage (31) est constituée de non-tissé qui est revêtu de matière plastique de préférence sur au moins une face.

6. Matériau d'emballage en forme de bande selon l'une des revendications précédentes, **caractérisé en ce que** le revêtement anti-adhérent (32, 35, 36) est positionné conformément aux exigences de l'objet à emballer (34).

7. Matériau d'emballage en forme de bande selon la revendication 6, **caractérisé en ce que** le revêtement anti-adhérent (32, 35, 36) est positionné pour recevoir une serviette hygiénique (34) avec ailettes latérales.

8. Matériau d'emballage en forme de bande selon la revendication 6, **caractérisé en ce que** le revêtement anti-adhérent (32, 35, 36) est positionné pour recevoir un pansement auto-adhésif.

9. Matériau d'emballage en forme de bande selon l'une des revendications précédentes, **caractérisé en ce que** la face de la bande de matériau d'emballage qui est opposée au revêtement anti-adhérent porte une impression.
